# EUROPÄISCHE PATENTSCHRIFT

(11) **EP 2 322 240 B1**
(45) Veröffentlichungstag und Bekanntmachung des Hinweises auf die Patenterteilung: **26.10.2016**
(21) Anmeldenummer: 10191056.0
(22) Anmeldetag: 12.11.2010
(51) Int. Cl.: A61M 16/04, A61F 2/20

(54) **Tracheostomiekanüle mit Fenster**
Tracheostomy cannula with window
Canule de trachéotomie dotée d'une fenêtre

(30) Priorität: 13.11.2009 DE 102009046703; 11.12.2009 DE 102009054573
(43) Veröffentlichungstag der Anmeldung: 18.05.2011
(73) Patentinhaber: TRACOE medical GmbH, 55268 Nieder-Olm (DE)
(72) Erfinder: Schnell, Ralf, 63500, Seligenstadt (DE)
(74) Vertreter: WSL Patentanwälte Partnerschaft mbB

(56) Entgegenhaltungen:
- WO-A1-2008/144589
- WO-A2-99/32169
- DE-A1- 10 109 935
- DE-A1- 19 543 169
- DE-A1-102006 035 887
- US-A- 4 852 565

## Beschreibung

Die vorliegende Erfindung betrifft eine Tracheostomiekanüle mit einem Kanülenrohr, wobei das Kanülenrohr eine Biegung mit einer radial innen und einer radial außen liegenden Seite des Kanülenrohrs und ein Rohrlumen und eine Rohrwandung und eine parallel zu der Rohrwandung in dem Rohrlumen verlaufende Achse und ein distales und ein proximales Ende und einen ersten Fensterbereich mit einer oder mehreren Öffnungen in der Rohrwandung aufweist.

Patienten mit einem Tracheostoma, bei denen der Kehlkopf teilweise oder vollständig erhalten ist, sind generell in der Lage zu sprechen. Allerdings muss dazu Luft von der Luftröhre beim Ausatmen in den Bereich des Kehlkopfs gelangen. Bei Tracheostomiekanülen mit einem geschlossenen Kanülenrohr ist dies nicht möglich, da die Luft durch das Kanülenrohr ausschließlich in die Luftröhre, die Bronchien und die Lunge und auf dem gleichen Weg zurück gelangt.

Um Patienten mit Kehlkopf dennoch ein Sprechen zu ermöglichen, werden Tracheostomiekanülen verwendet, bei denen das Kanülenrohr einen Fensterbereich aufweist. In diesem Bereich ist die Rohrwandung des Kanülenrohrs durch eine oder mehrere Öffnungen durchbrochen. Wird während des Ausatemvorgangs das Rohrlumen des Kanülenrohrs am proximalen Ende durch ein Ventil oder durch Abdecken mit einem Finger verschlossen, so kann die ausgeatmete Luft nicht durch das proximale Ende der Tracheostomiekanüle entweichen, sondern gelangt durch den Fensterbereich zum Kehlkopf, so dass der Patient sprechen kann.

Die Begriffe "distal" und "proximal" werden dabei aus der Sicht eines behandelnden Arztes verwendet, d.h. dass das proximale Ende der Tracheostomiekanüle bzw. des Kanülenrohrs außerhalb des Patientenkörpers liegt, während das distale Ende im eingebrachten Zustand der Tracheostomiekanüle im Inneren der Trachea liegt.

Obwohl Tracheostomiekanülen in der Regel individuell ausgewählt werden, kann es durch Verrutschen bei Bewegung oder Veränderung der anatomischen Verhältnisse, z. B. durch Wundheilung, vorkommen, dass der Fensterbereich nicht frei in der Trachea liegt, sondern an der Tracheawand oder umliegendem Gewebe anliegt. In diesen Fällen kann der Patient nicht oder nur mit sehr großem Kraftaufwand sprechen. Weiter besteht insbesondere bei frisch operierten Patienten die Gefahr, dass der Fensterbereich im Stomabereich anliegt und Luft in das Gewebe eindringt, wodurch es zur Ausbildung von Emphysemen kommt. WO 2008/144 589 beschreibt Tracheostomie Kanüle mit Fenster, US 4 852 565 ebenso.

Zur Lösung dieses Problems wurde beispielsweise in der DE 10 2006 035 887 vorgeschlagen, dass eine Tracheostomiekanüle eingesetzt wird, deren effektive Länge der Außenkanüle und/oder der Innenkanüle variabel ist. Durch Verwendung derartiger Tracheostomiekanülen ist es möglich, den Fensterbereich der Kanüle so zu positionieren, dass die Position an die jeweiligen anatomischen Verhältnisse optimal angepasst ist.

Gegenüber diesem Stand der Technik liegt der vorliegenden Erfindung die Aufgabe zugrunde, eine Tracheostomiekanüle mit einem Kanülenrohr zur Verfügung zu stellen, mit welcher auch dann, wenn sich die Position der Tracheostomiekanüle in der Trachea oder die anatomischen Verhältnisse verändern, ein Sprechen ermöglicht wird und ein Lufteintritt ins Gewebe verhindert wird.

Diese Aufgabe wird erfindungsgemäß dadurch gelöst, dass das Kanülenrohr axial versetzt zu dem ersten Fensterbereich mindestens einen weiteren Fensterbereich mit einer oder mehreren Öffnungen in der Rohrwandung aufweist.

Der weitere Fensterbereich ist zu dem ersten Fensterbereich axial versetzt an dem Kanülenrohr angeordnet, so dass ein Sprechen mit Hilfe der aus dem weiteren Fensterbereich entweichenden Luft möglich ist, wenn der erste Fensterbereich an der Trachea oder dem angrenzenden Gewebe anliegt oder umgekehrt. Weiterhin wird durch das Entweichen der Luft durch den freiliegenden Fensterbereich ein Eindringen der Luft ins Gewebe bei dem am Körpergewebe anliegenden Fensterbereich verhindert und damit das Risiko einer Emphysembildung verringert.

Die Fensterbereiche können unabhängig voneinander jeweils aus einer oder mehreren Öffnungen bestehen, wobei die mehreren Öffnungen einen kleineren Öffnungsquerschnitt aufweisen als eine einzelne Öffnung. Sofern mehrere Öffnungen vorhanden sind, sind diese zweckmäßigerweise auf einen gewissen Bereich, den Fensterbereich, beschränkt.

Bei bestimmten Ausführungsformen können der erste Fensterbereich und der mindestens -eine weitere Fensterbereich aneinander angrenzen, vorzugsweise sind der erste Fensterbereich und der mindestens eine weitere Fensterbereich voneinander beabstandet.

Die Fensterbereiche können unterschiedliche Ausmaße und Formen haben. Bevorzugt wird dabei jedoch ein Breitenmaß, gemessen in Umfangsrichtung des Kanülenrohrs, von etwa 1/4 bis 1/3 des Umfangs nicht überschritten. Weiterhin bevorzugt beträgt das axiale Ausmaß eines Fensterbereichs etwa 1/3 bis 1/2 des Umfangs des Kanülenrohrs. Sofern der Fensterbereich aus mehreren Öffnungen in der Rohrwandung besteht, kann der durch die Öffnungen abgedeckte Fensterbereich einschließlich der verbleibenden Zwischenräume größer sein, ist jedoch vorzugsweise immer so bemessen, dass der gesamte Fensterbereich ohne direkten Kontakt zum Körpergewebe in der Trachea freiliegen kann.

Bevorzugt ist der erste und/oder der weitere Fensterbereich mit einem Ventil oder einer einfachen Klappe versehen, das bzw. die die Öffnung(en) bedeckt und nur Luft nach außen treten lässt. Dieses Ventil oder die Klappe verhindert ein Eindringen von Fremdkörpern in die Tracheostomiekanüle und damit in die Trachea, indem es bzw. sie den Fensterbereich verschließt. Ein Öffnen des Fensterbereichs erfolgt nur dann, wenn durch entsprechenden Innendruck in dem Kanülenrohr die Klappe bzw. das Ventil aufgedrückt werden.

Die Tracheostomiekanüle kann bei bevorzugten Ausführungsformen für die perkutane Tracheostomie vorgesehen sein, wobei das Tracheostoma mit Hilfe einer Einführhilfe und der Tracheostomiekanüle selbst aufgeweitet bzw. offengehalten wird, so dass letztere durch das Tracheostoma in die Trachea eingeführt werden kann.

Bei bestimmten Ausführungsformen sind mehrere weitere Fensterbereiche in der Rohrwandung des Kanülenrohrs vorhanden, bevorzugt ist genau ein weiterer Fensterbereich in der Rohrwandung des Kanülenrohrs vorhanden. Je mehr Fensterbereiche vorhanden sind, desto problematischer kann das mögliche Eindringen von Sekreten und evtl. Festkörpern (z.B. Speisebrei) in die Tracheostomiekanüle werden. Andererseits sind insgesamt mindestens zwei Fensterbereiche für das Funktionieren der erfindungsgemäßen Tracheostomiekanüle erforderlich.

Bevorzugt ist der weitere Fensterbereich relativ zu dem ersten Fensterbereich in Umfangsrichtung des Kanülenrohrs um dessen Achse verschoben angeordnet.

Sofern einer der Fensterbereiche, beispielsweise durch Verrutschen der Tracheostomiekanüle in dem Tracheostoma bzw. der Trachea, an der Trachea oder dem umgebenden weiteren Körpergewebe anliegt, wird aufgrund der axial und in Umfangsrichtung versetzten Anordnung der Fensterbereiche mit hoher Wahrscheinlichkeit gewährleistet, dass ein oder mehrere Fensterbereich(e) freiliegen, wodurch weniger oder keine Luft an dem anliegenden Fensterbereichen in das Gewebe gelangt und gleichzeitig für den Patienten die Möglichkeit besteht zu sprechen.

Bevorzugt ist der erste Fensterbereich auf der radial außen liegenden Seite oder seitlich der Biegung des Kanülenrohrs angeordnet.

Die Biegung des Kanülenrohrs beschreibt bevorzugt einen Bogen mit einem eingeschlossenen Winkel von 100°, wobei Abweichungen von bis zu +/-20° umfasst sind. Wenn der erste Fensterbereich an der radial außen liegenden Seite der Biegung angeordnet ist, befindet sich der Mittelpunkt dieses Fensterbereichs vorzugsweise im Wesentlichen am Scheitelpunkt der Biegung, d.h. im Fall eines Kreisbogens bei etwa 50° ± 20° Abwinkelung gegenüber den Enden der Kanüle. Bei einer Tracheostomiekanüle in eingebrachtem Zustand kommt ein solcher erster Fensterbereich an der Oberseite des Kanülenrohrs im Bereich der Trachea zu liegen.

Besonders bevorzugt ist der weitere Fensterbereich auf der radial innen liegenden Seite der Biegung des Kanülenrohrs angeordnet.

Es hat sich gezeigt, dass diese Anordnung eines weiteren Fensterbereichs eine hohe Sicherheit dafür bietet, dass mindestens einer der Fensterbereiche freiliegt, um so das Sprachvermögen des Patienten zu erhalten, auch wenn einer der Fensterbereiche an Körpergewebe anliegt.

Bei einer bevorzugten Ausführungsform der Erfindung sind der erste und der weitere Fensterbereich an diametral einander gegenüberliegenden Seiten des Kanülenrohrs angeordnet.

Dies hat den Vorteil, dass auch bei einer in irgendeiner Richtung verschobenen Tracheostomiekanüle, bei welcher der erste Fensterbereich blockiert ist, ein Sprechen ermöglicht werden kann.

Gemäß einer bevorzugten Ausführungsform weist der erste Fensterbereich und/oder der zweite Fensterbereich eine Mehrzahl von Öffnungen in der Rohrwandung auf.

Die Öffnungen in den Fensterbereichen können unterschiedliche Größen und Formen haben. Bevorzugt sind bei dieser Ausführungsform die Öffnungen im Wesentlichen kreisförmig, wobei besonders bevorzugt der Durchmesser der Öffnungen zwischen 2 und 3 mm beträgt. Derart kleine Öffnungen verhindern insbesondere ein Eindringen von Fremdkörpern in das Kanülenrohr der Tracheostomiekanüle, wobei jedoch der Ausatemwiderstand nicht oder nur unwesentlich erhöht wird. Außerdem kann durch die kleinen Öffnungen verhindert werden, dass beim Absaugen von Schleim etc. aus der Kanüle der Sauger durch das Fenster aus der Kanüle austritt und die Tracheawand verletzt. Weiterhin bildet sich bei Fenstern mit kleineren Öffnungen weniger Granulationsgewebe an den Öffnungen, das sonst die Kanüle ggf. blockieren kann.

Bei einer bevorzugten Ausführungsform ist der weitere Fensterbereich relativ zu dem ersten Fensterbereich um einen Betrag von 0,3 cm bis 3 cm, bevorzugt 1 cm bis 2 cm, axial in Richtung distales Ende versetzt angeordnet. Es wird dabei von dem Abstand zwischen der am weitesten distal angeordneten Öffnung des ersten Fensterbereichs und der nächstliegenden proximal angeordneten Öffnung des weiteren Fensterbereichs ausgegangen.

Bei dieser Anordnung ist die Wahrscheinlichkeit besonders gering, dass beide Fensterbereiche an Körpergewebe anliegen.

Besonders bevorzugt beträgt die Summe der Öffnungsquerschnitte der einen oder mehreren Öffnungen des weiteren Fensterbereichs 1/10 bis 1/2, bevorzugt ein 1/5 bis 1/3, der Fläche des Querschnitts des Rohrlumens am proximalen Ende. Es hat sich gezeigt, dass bei einem Kanülenrohr, welches einen ersten Fensterbereich und mindestens einen weiteren Fensterbereich aufweist, der Öffnungsquerschnitt der Öffnungen des weiteren Fensterbereichs deutlich kleiner sein kann als der freie Kanülenquerschnitt.

Bei einer weiteren bevorzugten Ausführungsform beträgt der Öffnungsquerschnitt der einen oder mehreren Öffnungen des ersten und des weiteren Fensterbereiches jeweils 1/10 bis 1/3 der Fläche des Querschnitts des Rohrlumens am proximalen Ende.

Bedingt durch die kleineren Öffnungsflächen können die Fensterbereiche gemäß einer bevorzugten Ausführungsform weiter in Richtung distales Ende des Kanülenrohrs verlegt werden, im Vergleich zu herkömmlichen Tracheostomiekanülen mit Fensterbereich. Dadurch wird insbesondere eine Emphysembildung im Bereich des Stomas verhindert.

Besonders bevorzugt ist eine Ausführungsform der Erfindung, bei welcher der Querschnitt der einen oder mehreren Öffnungen des ersten Fensterbereichs im Wesentlichen gleich groß ist wie der Querschnitt der einen oder mehreren Öffnungen des weiteren Fensterbereichs. Es werden dadurch zwei gleichwertige Fensterbereiche zur Verfügung gestellt, die bei Blockieren eines der beiden Fensterbereiche ein Sprechen ohne erheblichen Kraftaufwand des Patienten ermöglichen.

Eine weitere bevorzugte Ausführungsform ist dadurch gekennzeichnet, dass die Tracheostomiekanüle eine Manschette aufweist, die axial in Richtung distales Ende versetzt relativ zu dem ersten und dem weiteren Fensterbereich angeordnet ist. Derartige Manschetten werden bei Tracheostomiekanülen verwendet, um zu verhindern, dass Schleim oder Festkörper, wie z.B. Essensreste, entlang des Kanülenrohrs außen an der Tracheostomiekanüle in die Trachea und schließlich in die Lunge gelangt. Insbesondere bei Dysphagiepatienten und Patienten mit künstlicher Beatmung wird eine Aspiration von Schleim oder Festkörpern dadurch verhindert.

Durch die Anordnung der Manschette distal zu den Fensterbereichen wird ein Sprechen des Patienten durch die Manschette nicht verhindert und gleichzeitig die Tracheostomiekanüle in eine im Wesentlichen zentrale Position innerhalb der Trachea gedrückt, wodurch ein Anliegen des ersten und des weiteren Fensterbereichs an der Trachea zusätzlich verhindert wird.

Die Tracheostomiekanüle weist weiter eine in das Kanülenrohr einsetzbare Innenkanüle mit einer Rohrwandung und einem Rohrlumen auf, wobei die Innenkanüle zu dem ersten und dem weiteren Fensterbereich des Kanülenrohrs korrespondierende innere Fensterbereiche mit einer oder mehreren Öffnungen in der Rohrwandung aufweist, die in axialer Richtung jeweils mindestens teilweise mit dem ersten oder dem weiteren Fensterbereich des Kanülenrohrs überlappen.

Der Vorteil einer Tracheostomiekanüle mit einem Kanülenrohr und einer Innenkanüle besteht darin, dass, wenn ein Sprechen nicht erforderlich ist, die Innenkanüle mit einer Rohrwandung mit Fensterbereichen durch eine Innenkanüle ohne Fensterbereiche ausgetauscht werden kann. Außerdem kann eine Reinigung der Innenkanüle und damit Entfernung von evtl. in der Tracheostomiekanüle vorhandenen Verunreinigungen leichter dadurch erfolgen, dass die Innenkanüle entnommen wird, wobei das Kanülenrohr in dem Tracheostoma verbleibt. Derartige Innenkanülen sind so gestaltet, dass sie in das Rohrlumen des Kanülenrohrs eingepasst sind und insbesondere im distalen Bereich dicht mit dem Kanülenrohr abschließen. Insbesondere dadurch kann ein Hinablaufen von durch die Fensterbereiche eindringenden Sekrets innerhalb des Kanülenrohrs in die Trachea und schließlich in die Lunge verhindert werden.

Bevorzugt sind die Fensterbereiche des Kanülenrohrs mit den einen oder mehreren Öffnungen in der Rohrwandung so angeordnet, dass mindestens 50%, bevorzugt mindestens 70%, besonders bevorzugt mindestens 80% der Öffnungsfläche der inneren Fensterbereiche mit den Öffnungsflächen der Fensterbereiche in dem Kanülenrohr überlappt.

Bevorzugt sind die Öffnungsquerschnitte der korrespondierenden inneren Fensterbereiche größer als die Öffnungsquerschnitte der jeweiligen Fensterbereiche in dem Kanülenrohr.

Bevorzugt liegt die Innenkanüle mit Fensterbereich im Rohrlumen eng an der Innenwand der Rohrwandung des Kanülenrohrs an. Dadurch wird verhindert, dass Luft zwischen der Rohrwandung der Innenkanüle und der Rohrwandung des Kanülenrohrs entlangströmt und dadurch möglicherweise den Atemwiderstand erhöht.

Gemäß einer besonders bevorzugten Ausführungsform weisen die korrespondierenden inneren Fensterbereiche der Innenkanüle jeweils genau eine Öffnung in der Rohrwandung auf. Bevorzugt besteht dadurch die Möglichkeit in Kombination mit einem Kanülenrohr mit Fensterbereichen mit mehreren Öffnungen in der Rohrwand eine Tracheostomiekanüle bereitzustellen, bei der eine möglichst kleine Öffnungsfläche in dem Kanülenrohr, die durch mehrere Öffnungen mit geringen Durchmessern bereitgestellt wird, auch dann ein Sprechen mit geringem Luftwiderstand ermöglicht, wenn die korrespondierenden inneren Fensterbereiche der Innenkanüle nicht exakt mit den Fensterbereichen des Kanülenrohrs übereinstimmen. Dies hat insbesondere Vorteile bei der Herstellung der Tracheostomiekanülen, denn es können auf diese Weise Tracheostomiekanülen mit einem Kanülenrohr mit identischem Durchmesser des Rohrlumens und identischer Länge aber (leicht) unterschiedlichen Positionen der Fensterbereiche zusammen mit einer einzigen Art von Innenkanüle verwendet werden.

Gemäß einer weiteren bevorzugten Ausführungsform weist ein innerer Fensterbereich der Innenkanüle eine Öffnung auf und ein anderer innerer Fensterbereich mehrere Öffnungen, die mit den Öffnungen des Fensterbereichs in dem Kanülenrohr korrespondieren.

Weitere Vorteile, Merkmale und Anwendungsmöglichkeiten der vorliegenden Erfindung werden deutlich anhand der folgenden Beschreibung von bevorzugten Ausführungsformen und der dazugehörigen Figuren. Es zeigen:
- Fig. 1: eine perspektivische Darstellung einer Tracheostomiekanüle mit Kanülenrohr mit Aufsicht auf die radial außen liegende Seite der Biegung,
- Fig. 2: eine perspektivische Ansicht einer Tracheostomiekanüle mit Innenkanüle, in seitlicher Aufsicht auf die Biegung des Kanülenrohrs und
- Fig. 3: eine Innenkanüle, wie sie in Fig. 2 in das Kanülenrohr eingesetzt sein kann.

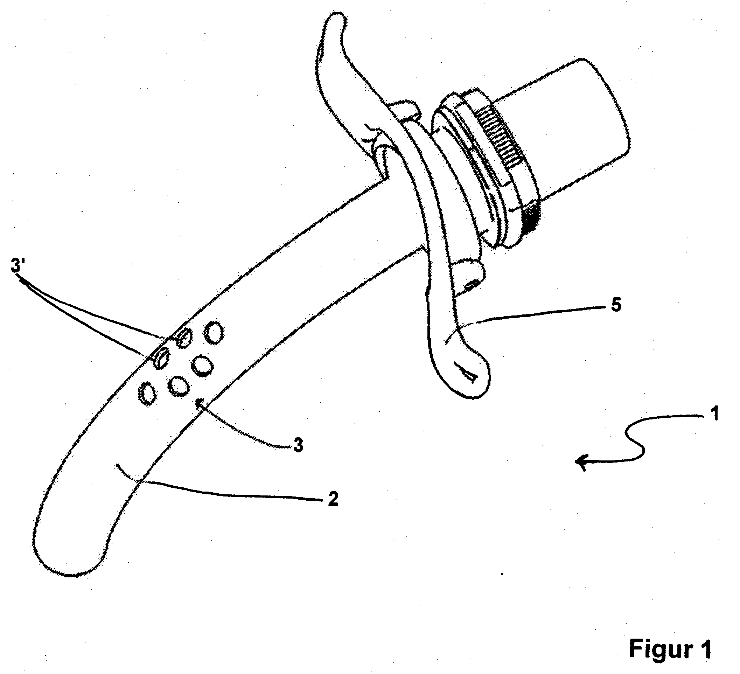

In Fig. 1 ist eine mit 1 bezeichnete Tracheostomiekanüle dargestellt. Diese weist ein Kanülenrohr 2 auf, welches wiederum einen Fensterbereich 3 an der radial außen liegenden Seite der Biegung der Rohrwandung aufweist. Der Fensterbereich 3 besteht aus insgesamt sechs Öffnungen 3'. Zu dem Fensterbereich 3 ist ein weiterer Fensterbereich 4 vorhanden, der axial versetzt zu den Fensterbereich 3 und in Umfangsrichtung des Kanülenrohrs so um die Achse verschoben angeordnet ist, dass er in der in Fig. 1 gewählten Perspektive nicht sichtbar ist. An dem proximalen Ende des Kanülenrohrs 2 ist ein Schild 5 angebracht, der zur Anlage an die ein Tracheostoma umgebende Halsoberfläche eines Patienten vorgesehen ist.

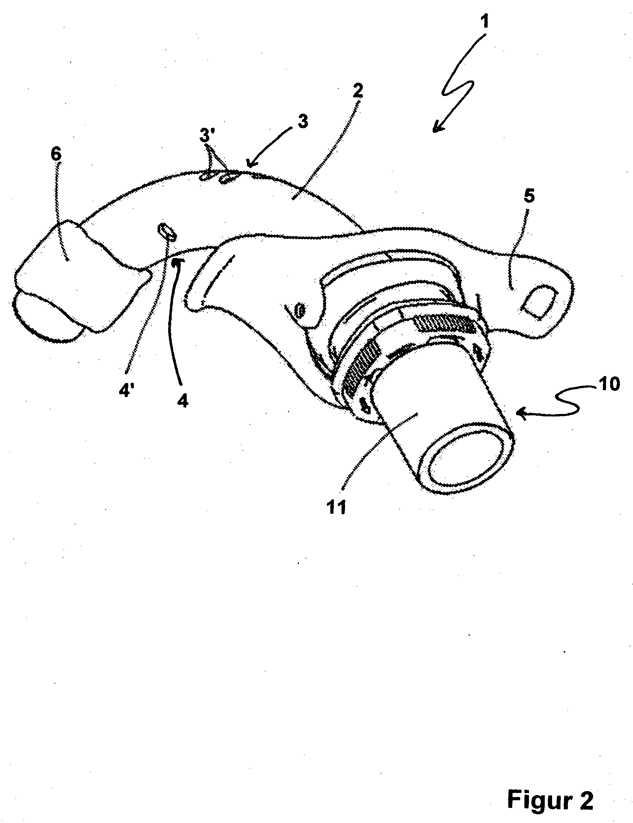

In Fig. 2 ist eine weitere Ausführungsform einer erfindungsgemäßen Tracheostomiekanüle 1 dargestellt. Die Tracheostomiekanüle weist ein Kanülenrohr 2 auf, mit einem ersten Fensterbereich 3 und einem weiteren Fensterbereich 4. Der erste Fensterbereich besteht aus mehreren Öffnungen, von denen drei Öffnungen 3' dargestellt sind. Der weitere Fensterbereich 4 ist axial versetzt zu dem ersten Fensterbereich angeordnet und befindet sich auf der radial innen liegenden Seite der Biegung des Kanülenrohrs 2. Der weitere Fensterbereich besteht aus zwei Öffnungen, von denen eine Öffnung 4' dargestellt ist.

Das Schild 5 dient wie bei der in Fig. 1 dargestellten Ausführungsform der Anlage an die ein Tracheostoma umgebende Halsoberfläche eines Patienten. Es befindet sich an dem proximalen Ende des Kanülenrohrs 3 der Tracheostomiekanüle 1.

Distal zu dem ersten Fensterbereich 3 und dem zweiten Fensterbereich 4 ist eine Manschette angeordnet. Diese Manschette ist über einen nicht dargestellten Ballon und Füllschlauch nach dem Einführen der Tracheostomiekanüle durch ein Tracheostoma in die Trachea aufpumpbar. Die Manschette liegt dabei mit leichtem Überdruck an der Tracheawand an.

An dem proximalen Ende der Tracheostomiekanüle 1 ist ein Teil einer in das Kanülenrohr 2 eingeschobenen Innenkanüle 10 erkennbar. Bei diesem Teil handelt es sich um den Konnektor 11, mit dessen Hilfe beispielsweise ein Sprechventil, das den Einlass von Luft in die Tracheostomiekanüle ermöglicht, aber das Ausatmen durch die Öffnung der Tracheostomiekanüle verhindert, anbringbar ist.

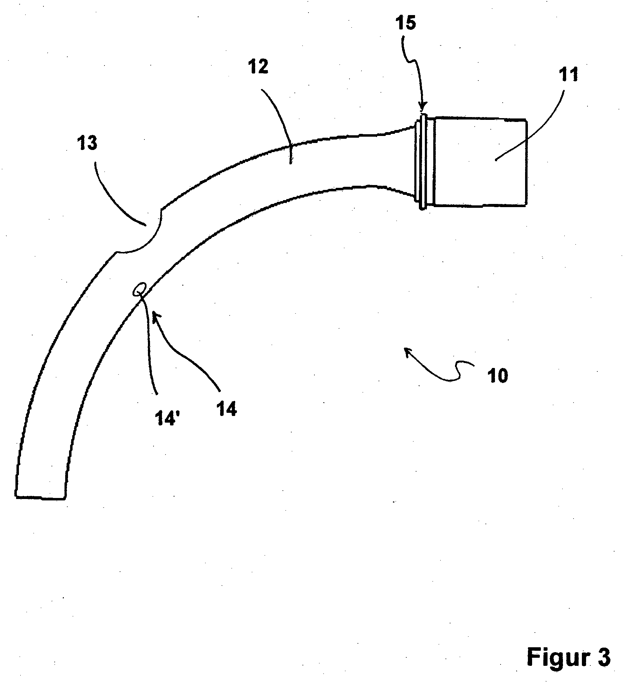

In Fig. 3 ist eine derartige Innenkanüle 10 dargestellt, wie sie bei der in Fig. 2 dargestellten Tracheostomiekanüle verwendet werden kann.

Die Innenkanüle verfügt über einen Konnektor 11, mit dem ein Sprechventil befestigbar ist. Weiter verfügt die Innenkanüle über ein Rohr 12 mit Rohrwandung und Rohrlumen, wobei in die Rohrwandung ein erster Fensterbereich 13 und ein zweiter Fensterbereich 14 eingebracht sind. Der erste Fensterbereich 13 umfasst eine einzige Öffnung. Beim Einbringen der Innenkanüle in ein Kanülenrohr, wie es in Fig. 2 dargestellt ist, korrespondiert der innere Fensterbereich 13 mit dem Fensterbereich 3 des Kanülenrohrs. Der zweite Fensterbereich umfasst genau zwei Öffnungen 14', von denen eine dargestellt ist. Die Öffnungen 14' korrespondieren in eingebrachtem Zustand der Innenkanüle 10 in das Kanülenrohr 1 mit den Öffnungen 4' des in Fig. 2 dargestellten Kanülenrohrs 1, wobei die Öffnungen 14' in der Innenkanüle 10 geringfügig größer sind, als die Öffnungen 4' in dem Kanülenrohr 1.

Der Abschnitt 15 stellt einen Adapter dar, mit welchem die Innenkanüle an einem Schild, welches sich an dem proximalen Ende eines Kanülenrohrs befindet, befestigbar ist.

Für Zwecke der ursprünglichen Offenbarung wird darauf hingewiesen, dass sämtliche Merkmale, wie sie sich aus der vorliegenden Beschreibung, den Zeichnungen und den abhängigen Ansprüchen für einen Fachmann erschließen, auch wenn sie konkret nur im Zusammenhang mit bestimmten weiteren Merkmalen beschrieben wurden, sowohl einzeln als auch in beliebigen Zusammenstellungen mit anderen der hier offenbarten Merkmale oder Merkmalsgruppen kombinierbar sind, soweit dies nicht ausdrücklich ausgeschlossen wurde oder technische Gegebenheiten derartigen Kombinationen unmöglich oder sinnlos machen. Dies gilt entsprechend auch für jeweils mehrere in einem abhängigen Anspruch aufgezählte Merkmale, die jeweils individuell verwirklicht werden können, sofern sie sich nicht unmittelbar aus technischen Gründen bedingen. Auf die umfassende, explizite Darstellung sämtlicher denkbarer Merkmalskombinationen wird hier nur der Kürze und der Lesbarkeit der Beschreibung wegen verzichtet.

## Patentansprüche

1. Tracheostomiekanüle (1) mit einem Kanülenrohr (2), wobei das Kanülenrohr eine Biegung mit einer radial innen und einer radial außen liegenden Seite des Kanülenrohres und ein Rohrtumen und eine Rohrwandung und eine parallel zu der Rohrwandung in dem Rohrlumen verlaufende Achse und ein distales und ein proximales Ende und einen ersten Fensterbereich (3) mit einer oder mehreren Öffnungen (3') in der Rohrwandung aufweist, wobei das Kanülenrohr axial versetzt zu dem ersten Fensterbereich mindestens einen weiteren Fensterbereich (4) mit einer oder mehreren Öffnungen (4') in der Rohrwandung aufweist und die Tracheostomiekanüle eine Innenkanüle (10) mit einer Rohrwandung und einem Rohrlumen aufweist, die in das Kanülenrohr einsetzbar ist, **dadurch gekennzeichnet, dass** die Innenkanüle zu dem ersten und dem weiteren Fensterbereich des Kanülenrohrs korrespondierende Innere Fensterbereiche (13, 14) mit einer oder mehreren Öffnungen (13', 14') in der Rohrwandung aufweist, die In axiale Richtung jeweils mindestens teilweise mit dem ersten oder dem weiteren Fensterbereich des Kanülenrohrs überlappen.

2. Tracheostomlekanüle (1) gemäß Anspruch 1, **dadurch gekennzeichnet, dass** der weitere Fensterbereich (4) relativ zu dem ersten Fensterbereich (3) In Umfangsrichtung des Kanülenrohrs um dessen Achse verschoben angeordnet ist.

3. Tracheostomiekanüle (1) gemäß einem der vorangehenden Ansprüche, **dadurch gekennzeichnet, dass** der erste Fensterbereich (3) auf der radial außen liegenden Seite oder seitlich der Biegung des Kanülenrohrs angeordnet ist.

4. Tracheostomiekanüle (1) gemäß einem der vorangehenden Ansprüche, **dadurch gekennzeichnet dass** der weitere Fensterbereich (4) auf der radial innen liegenden Seite der Biegung des Kanülenrohrs angeordnet ist.

5. Tracheostomiekanüle (1) gemäß einem der vorangehenden Ansprüche, **dadurch gekennzeichnet, dass** der erste und der weitere Fensterbereich (3, 4) an diametral einander gegenüberliegenden Seiten des Kanülenrohrs angeordnet sind.

6. Tracheostomiekanüle (1) gemäß einem der vorangehenden Ansprüche, **dadurch gekennzeichnet, dass** der erste Fensterbereich (3) und/oder der zweite Fensterbereich (4) eine Mehrzahl von Öffnungen (3', 4') in der Rohrwandung aufweisen.

7. Tracheostomiekanüle (1) nach Anspruch 6, **dadurch gekennzeichnet, dass** die Öffnungen (3', 4') im Wesentlichen kreisförmig sind und bevorzugt der Durchmesser der Öffnungen zwischen 2 und 3 mm beiträgt

8. Tracheostomiekanüle (1) gemäß einem der vorangehenden Ansprüche, **dadurch gekennzeichnet, dass** die Fensterbereiche (3, 4) ein Breitenmaß aufweisen, welches gemessen in Umfangsrichtung des Kanülenrohrs etwa 1/3 des Umfangs nicht überschreitet und/oder ein Fensterbereich ein axiales Ausmaß aufweist, das etwa 1/3 bis 1/2 des Umfangs des Kanülenrohrs beträgt.

9. Tracheostomiekanüle (1) gemäß einem der vorangehenden Ansprüche, **dadurch gekennzeichnet, dass** der weitere Fensterbereich (4) relativ zu dem ersten Fensterbereich (3) um einen Betrag von 0,3 cm bis 3 cm, bevorzugt 1 cm bis 2 cm, axial In Richtung distales Ende versetzt angeordnet ist.

10. Tracheostomiekanüle (1) gemäß einem der vorangehenden Ansprüche, **dadurch gekennzeichnet, dass** die Summe der Öffnungsquerschnitte der einen oder mehreren Öffnungen (4') des weiteren Fensterbereichs 1/10 bis 1/2, bevorzugt 1/5 bis 1/3, der Fläche des Querschnitts des Rohrlumens am proximalen Ende beträgt und/oder der Öffnungsquerschnitt der einen oder mehreren Öffnungen (3', 4') des ersten und des weiteren Fensterbereichs (3, 4) jeweils 1/10 bis 1/3 der Fläche des Querschnitts des Rohrlumens am proximalen Ende beträgt

11. Tracheostomiekanüle (1) gemäß einem der vorangehenden Ansprüche, **dadurch gekennzeichnet, dass** der Querschnitt der einen oder mehreren Öffnungen (3') des ersten Fensterbereichs (3) im Wesentlichen gleich groß ist wie der Querschnitt der einen oder mehreren Öffnungen (4') des weiteren Fensterbereichs (4).

12. Tracheostomlekanüfe (1) gemäß einem der vorangehenden Ansprüche, **dadurch gekennzeichnet, dass** die Tracheostomiekanüle eine Manschette (6) aufweist, die axial In Richtung distales Ende versetzt relativ zu dem ersten und dem weiteren Fensterbereich angeordnet ist.

13. Tracheostomiekanüle (1) gemäß einem der vorangehenden Ansprüche, **dadurch gekennzeichnet, dass** die Fensterbereiche (3, 4) des Kanülenrohrs (2) mit den einen oder mehreren Öffnungen in der Rohrwandung so angeordnet sind, dass mindestens 50%, bevorzugt mindestens 70%, besonders bevorzugt mindestens 80% der Öffnungsfläche der inneren Fensterbereiche (13, 14) mit den Öffnungsflächen der Fensterbereiche (3, 4) in dem Kanülenrohr überlappt.

14. Tracheostomiekanüle gemäß einem der vorangehenden Ansprüche, **dadurch gekennzeichnet, dass** die korrespondierenden inneren Fensterbereiche (13, 14) der Innenkanüle (10) jeweils genau eine Öffnung in der Rohrwandung aufweisen oder dass ein innerer Fensterbereich (13, 14) eine Öffnung und ein anderer innerer Fensterbereich (13, 14) mehrere Öffnungen aufweist, die mit den Öffnungen des Fensterbereichs in dem Kanülenrohr korrespondieren.

## Claims

1. A tracheostomy cannula (1) comprising a cannula tube (2), wherein the cannula tube has a bend with a radially inward and a radially outward side of the cannula tube and a tube lumen and a tube wall and an axis extending parallel to the tube wall in the tube lumen and a distal and a proximal end and a first window region (3) having one or more openings (3') in the tube wall, wherein the cannula tube has in axially displaced relationship with the first window region at least one further window region (4) having one or more openings (4') in the tube wall, and that the tracheostomy cannula has an inner cannula (10) having a tube wall and a tube lumen, which can be inserted into the cannula tube, **characterised in that** the inner cannula has inner window regions (13, 14) corresponding to the first and the further window regions of the cannula tube and having one or more openings (13', 14') in the tube wall which at least partially overlap in the axial direction with the first or the further window region of the cannula tube.

2. A tracheostomy cannula (1) as set forth in claim 1 **characterised in that** the further window region (4) is arranged displaced relative to the first window region (3) in the peripheral direction of the cannula tube about the axis thereof.

3. A tracheostomy cannula (1) as set forth in one of the preceding claims **characterised in that** the first window region (3) is arranged on the radially outward side or laterally of the bend in the cannula tube.

4. A tracheostomy cannula (1) as set forth in one of the preceding claims **characterised in that** the further window region (4) is arranged on the radially inward side of the bend in the cannula tube.

5. A tracheostomy cannula (1) as set forth in one of the preceding claims **characterised in that** the first and the further window regions (3, 4) are arranged at diametrally mutually opposite sides of the cannula tube.

6. A tracheostomy cannula (1) as set forth in one of the preceding claims **characterised in that** the first window region (3) and/or the second window region (4) have a plurality of openings (3', 4') in the tube wall.

7. A tracheostomy cannula (1) as set forth in claim 6 **characterised in that** the openings (3', 4') are substantially circular and whereby preferably the diameter of the openings is between 2 and 3 mm.

8. A tracheostomy cannula (1) as set forth in one of the preceding claims **characterised in that** the window regions (3, 4) have a width dimension, measured in the peripheral direction of the cannula tube, not exceeding about 1/3 of the periphery, and/or the axial dimension of a window region is between about 1/3 and 1/2 of the periphery of the cannula tube.

9. A tracheostomy cannula (1) as set forth in one of the preceding claims **characterised in that** the further window region (4) is arranged displaced relative to the first window region (3) axially in the direction of the distal end by an amount of between 0.3 cm and 3 cm, preferably between 1 cm and 2 cm.

10. A tracheostomy cannula (1) as set forth in one of the preceding claims **characterised in that** the sum of the cross-sections of the one or more openings (4') of the further window region is between 1/10 and 1/2, preferably between 1/5 and 1/3, of the area of the cross-section of the tube lumen at the proximal end, and/or **in that** the opening cross-section of the one or more openings (3', 4') of the first and the further window region (3, 4) is respectively 1/10 to 1/3 of the area of the cross-section of the tube lumen at the proximal end.

11. A tracheostomy cannula (1) as set forth in one of the preceding claims **characterised in that** the cross-section of the one or more openings (3') of the first window region (3) is of substantially the same size as the cross-section of the one or more openings (4') of the further window region (4).

12. A tracheostomy cannula (1) as set forth in one of the preceding claims **characterised in that** the tracheostomy cannula has a cuff (6) which is arranged displaced axially in the direction of the distal end relative to the first and the further window regions.

13. A tracheostomy cannula (1) as set forth in one of the preceding claims **characterised in that** the window regions (3, 4) of the cannula tube (2) with the one or more openings in the tube wall are so arranged that at least 50 % and preferably at least 70 % and particularly preferably at least 80 % of the opening areas of the inner window regions (13, 14) overlaps with the opening areas of the window regions (3, 4) in the cannula tube.

14. A tracheostomy cannula as set forth in one of the preceding claims **characterised in that** the corresponding inner window regions (13, 14) of the inner cannula (10) each have precisely one opening in the tube wall, or an inner window region (13, 14) has an opening and another inner window region (13, 14) has a plurality of openings corresponding to the openings of the window region in the cannula tube.

## Revendications

1. Canule de trachéostomie (1) dotée d'un tube de canule (2), dans laquelle le tube de canule présente une courbure avec un côté situé radialement à l'intérieur et un côté situé radialement à l'extérieur, une lumière, une paroi de tube, un axe s'étendant parallèlement à la paroi de tube dans la lumière, une extrémité distale, une extrémité proximale et une première zone de fenêtre (3) comportant une ou plusieurs ouvertures (3') dans la paroi de tube, le tube de canule comportant au moins une autre zone de fenêtre (4) avec une ou plusieurs ouvertures (4') dans la paroi de tube et décalée axialement par rapport à la première zone de fenêtre, et ladite canule de trachéostomie présentant une canule interne (10) avec une paroi de tube et une lumière de tube pouvant être insérée dans le tube de canule, **caractérisée en ce que** la canule interne présente des zones de fenêtre internes (13, 14) correspondant à la première et à l'autre zone de fenêtre du tube de canule et comportant dans la paroi de tube une ou plusieurs ouvertures (13', 14') qui chevauchent, du moins en partie, dans le sens axial, la première ou l'autre zone de fenêtre du tube de canule.

2. Canule de trachéostomie (1) selon la revendication 1, **caractérisée en ce que** l'autre zone de fenêtre (4) est décalée par rapport à la première zone de fenêtre (3), dans le sens périphérique du tube de canule, autour de l'axe de celle-ci.

3. Canule de trachéostomie (1) selon l'une quelconque des revendications précédentes, **caractérisée en ce que** la première zone de fenêtre (3) est disposée sur le côté radialement à l'extérieur de la courbure du tube de canule ou latéralement à celle-ci.

4. Canule de trachéostomie (1) selon l'une quelconque des revendications précédentes, **caractérisée en ce que** l'autre zone de fenêtre (4) est disposée sur le côté radialement à l'intérieur de la courbure du tube de canule.

5. Canule de trachéostomie (1) selon l'une quelconque des revendications précédentes, **caractérisée en ce que** la première et l'autre zone de fenêtre (3,4) sont disposées sur des côtés diamétralement opposés du tube de canule.

6. Canule de trachéostomie (1) selon l'une quelconque des revendications précédentes, **caractérisée en ce que** la première zone de fenêtre (3) et/ou la deuxième zone de fenêtre (4) présentent une pluralité d'ouvertures (3', 4') dans la paroi de tube.

7. Canule de trachéostomie (1) selon la revendication 6, **caractérisée en ce que** les ouvertures (3', 4') sont essentiellement circulaires et le diamètre des ouvertures mesure, de préférence, entre 2 et 3 mm.

8. Canule de trachéostomie (1) selon l'une quelconque des revendications précédentes, **caractérisée en ce que** les zones de fenêtre (3, 4) ont une largeur, mesurée dans le sens périphérique du tube de canule, ne dépassant pas 1/3 environ de la circonférence et/ou une zone de fenêtre a une étendue axiale égale à 1/3 à 1/2 environ de la circonférence du tube de canule.

9. Canule de trachéostomie (1) selon l'une quelconque des revendications précédentes, **caractérisée en ce que** l'autre zone de fenêtre (4) est décalée de 0,3 cm à 3 cm, de préférence de 1 cm à 2 cm, axialement en direction de l'extrémité distale, par rapport à la première zone de fenêtre (3).

10. Canule de trachéostomie (1) selon l'une quelconque des revendications précédentes, **caractérisée en ce que** le total des sections de l'une ou plusieurs ouvertures (4') de l'autre zone de fenêtre mesure de 1/10^{ème} à 1/2, de préférence de 1/5^{ème} à 1/3 de la surface de section transversale de la lumière du tube au niveau de l'extrémité proximale et/ou la section de l'une ou plusieurs ouvertures (3', 4') de la première et de l'autre zone de fenêtre (3, 4) mesure respectivement 1/10^{ème} à 1/3 de la surface de section transversale de la lumière de tube au niveau l'extrémité proximale.

11. Canule de trachéostomie (1) selon l'une quelconque des revendications précédentes, **caractérisée en ce que** la section transversale de l'une ou plusieurs ouvertures de la première zone de fenêtre (3) est essentiellement égale à la section transversale de l'une ou plusieurs ouvertures (4') de l'autre zone de fenêtre (4).

12. Canule de trachéostomie (1) selon l'une quelconque des revendications précédentes, **caractérisée en ce qu'**elle présente un manchon (6) décalé axialement, en direction de l'extrémité distale, par rapport à la première et à l'autre zone de fenêtre.

13. Canule de trachéostomie (1) selon l'une quelconque des revendications précédentes, **caractérisée en ce que** les zones de fenêtre (3, 4) du tube de canule (2) comportant une ou plusieurs ouvertures dans la paroi de tube, sont disposées de telle sorte qu'au moins 50 %, de préférence au moins 70 %, de manière particulièrement préférée au moins 80 %, de la surface d'ouverture des zones de fenêtre (13, 14) internes soient recouvertes par les surfaces d'ouverture des zones de fenêtre (3, 4) dans le tube de canule.

14. Canule de trachéostomie (1) selon l'une quelconque des revendications précédentes, **caractérisée en ce que** les zones de fenêtre internes correspondantes (13, 14) de la canule interne (10) présentent chacune précisément une ouverture dans la paroi de tube ou **en ce qu'**une zone de fenêtre interne (13, 14) présente une ouverture et une autre zone de fenêtre interne (13, 14) plusieurs ouvertures, qui correspondent aux ouvertures de la zone de fenêtre dans le tube de canule.
